# EUROPEAN PATENT APPLICATION

(11) **EP 4 571 768 A1**
(43) Date of publication of application: **18.06.2025**
(21) Application number: 23217210.6
(22) Date of filing: 15.12.2023
(51) Int. Cl.: G16H 40/20, G06Q 10/06

(54) **A GRAPHICAL USER INTERFACE SYSTEM FOR PROVIDING AN INTERACTIVE REPRESENTATION OF A HEALTH CARE FACILITY**

(71) Applicant: Baxter Medical Systems GmbH + Co. KG, 07318 Saalfeld (DE)
(72) Inventor: Schmotz, Christoph, 07318 Saalfeld (DE); Sylvester, Jordan, 07318 Saalfeld (DE); Furgoni, Stefano, 07318 Saalfeld (DE)
(74) Representative: Reddie & Grose LLP

(57) **Abstract**

A graphical user interface system for providing an interactive representation of a health care facility, the system comprising: a computing system comprising a memory including resource data of resources of a health care facility, the resources including a plurality of devices; a graphical user interface in communication with the computing system; and a three-dimensional graphical representation of the health care facility displayable by the graphical user interface for interactively managing the resources of the health care facility, wherein the three-dimensional graphical representation is configured to provide an output related to at least one device of the plurality of devices, the output being interactable by a user for performing at least one action related to the at least one device.

## Description

### FIELD OF THE INVENTION

The present invention relates to a graphical user interface system and in particular a graphical user interface system for providing an interactive representation of a health care facility. The present invention also relates to a method for providing an interactive representation of a health care facility.

### BACKGROUND OF THE INVENTION

Environments such as health care facilities or hospitals are required to manage many resources across various care settings, and doing so can require communication between various health care practitioners across various communications systems, which can be inefficient.

Limitations and inefficiencies can arise in complex workflows through the lack management of such resources across various care settings. Resources include management of various aspects of patient care and health care records, devices throughout the health care facility, and scheduling of health care facilities, practitioners, and patients. Interaction with such resources is typically performed manually with each resource, through communication with health care practitioners, patients, or physical interaction with devices or scheduling systems.

### SUMMARY OF THE INVENTION

The inventors of the present disclosure have appreciated the need for a centralized control system in environments such as health care facilities. The inventors have also appreciated the need for a user interface capable of synchronizing different workflows across care settings, providing the opportunity for connectivity between workflows in a smart way through the provision of such a control center. In this way, efficiency of complex workflows in environments such as health care facilities can be improved.

Arrangements of the present disclosure provide a three-dimensional graphical representation enabling navigation and interactive management of a complex workflow in an environment such as a health care facility. Throughout this disclosure, reference to a health care facility will be made, but it will be appreciated that the three-dimensional graphical representation described will be applicable to any suitable environment.

The three-dimensional graphical representation may allow for varying depth of detail throughout the health care facility depending on the required level of detail to be accessed. For example, a health care facility may be a surgical facility including a plurality of operating rooms (ORs). It will be appreciated that systems described herein may also be non-exhaustively applied to other health care facilities including an intensive care unit (ICU), outpatient facility, or emergency department. As will be discussed in more detail below, the graphical representation may provide an interactive map providing a user with the ability to zoom in or out of particular areas of the surgical department, showing varying levels of detail of the surgical facility which may be interactable by a user through the displayed graphical representation.

Arrangements of the present disclosure provide for such an interactive representation of a health care facility in which health care resources, patients, and health care practitioners can be tracked, viewed, and interacted with by a user through the graphical representation. The interactive representation may therefore provide a real time representation of traffic within the health care facility. An interactive representation may be a representation configured to receive inputs which may affect the hospital environment. Interactive management of a health care facility may allow for improvements in complex workflows. It may be particularly advantageous to provide interactive management of health care resources including devices in a health care facility. As will be described in more detail, by monitoring and interactively managing health care resources such as devices, issues giving rise to inefficiencies may be identified and resolved through the interactive management, improving the complex workflows of the health care facility.

According to an aspect of the present disclosure, there is provided a graphical user interface, GUI, system for providing an interactive representation of a health care facility, the system comprising: a computing system comprising a memory including resource data of resources of a health care facility, the resources including a plurality of devices; a graphical user interface in communication with the computing system; and a three-dimensional graphical representation of the health care facility displayable by the graphical user interface for interactively managing the resources of the health care facility, wherein the three-dimensional graphical representation is configured to provide an output related to at least one device of the plurality of devices, the output being interactable by a user for performing at least one action related to the at least one device.

The graphical representation may be displayable, for example, through the display of a graphical user interface on a display or monitor. Such a display or monitor may comprise any suitable computing device such as a computer, laptop, or portable computing device, and may be particularly advantageous when displayed on a touch screen display such as a portable mobile device or tablet configured to receive touch inputs for interacting with the graphical representation of the health care facility.

In some embodiments, the plurality of devices comprise one or more of: a display; a camera; a surgical table; an instrument cart; a surgical light; surgical equipment; and diagnostic equipment. It will be appreciated that this is a non-exhaustive list of the devices which may be displayed and interacted with through the graphical representation.

In some embodiments, the output related to the at least one device comprises one or more of: a status of the at least one device; an alarm related to the at least one device; and a video stream from the at least one device, the output being configured to be displayed by the three-dimensional graphical representation. The video stream may be a live feed output, for example, in real time from a device such as a camera or surgical device. The graphical user interface may be configured to display the video stream of the device, which may be in response to a user input selecting the relevant device. The video stream may then be monitored by a user of the system, and may be recorded by the computing system and stored in a memory of the computing system. The video stream may be received and/or processed by a software application integrated with the computing system.

In some embodiments, the system, for example the computing system, may be configured to track or monitor the location or position of devices in or related to the health care facility. Such tracking may be implemented by any suitable means, for example using a real-time locating system (RTLS), which may in some examples may implement ultra wide band receivers and transmitters, or beacons installed into or otherwise related to the plurality of devices. In this way, the location of devices in or related to the health care facility may be monitored and interactively managed in real time through the graphical representation. In some embodiments, the computing system provides this tracking functionality. In some embodiments, the computing system is configured to communicate with, such as receive data and/or information from, a locating system such as an RTLS.

In some embodiments, the status of the device comprises a location status of one or more components of the device, the location status indicating whether the one or more components of the device are located within a predetermined proximity of the device. That is, similarly to the location tracking of the devices, individual components of devices may also be tracked in the health care facility and viewed through the graphical representation. Statuses may also be displayed in relation to areas or departments of the health care facility, patients, and/or health care practitioners. Status may be displayed as colours, where colours may non-exhaustively correspond to whether a room or hospital bed is in use, the progress of a patient through the health care facility, the condition of a patient, or the availability of a health care practitioner.

In some embodiments, the status may also show data related to the functionality of a particular device. For example, where the device comprises diagnostic equipment or a diagnostic device, the status may indicate an output of the device such as vital signs readings of a patient being monitored by the diagnostic device. The status may also indicate whether or not the particular device is functioning properly. For example, the status may indicate that a device is not properly functioning and requires maintenance.

The status of the device may be displayable by the three-dimensional graphical representation, for example in the form of a notification or pop-up notification displayed by the graphical representation. Such a status may appear in response to a user input selecting a particular device displayed by the graphical representation through the graphical user input. Such a selection may be performed by a user via a touch input on a display or monitor on which the graphical representation is displayed. Alternatively or in addition, the status of a device may appear automatically in response to a user selecting a particular view or level of detail for the graphical representation, such as by selecting a particular OR in a surgical facility, the graphical representation then displaying the selected OR including one or more devices within the OR, and one or more corresponding statuses of devices within the selected OR.

In some embodiments, the alarm related to the device provides an indication that a component of the device is not located within the predetermined proximity of the device, and wherein the computing system determines the component as a missing component. As described, the location of components of a device may be tracked. The computing system may have stored in its memory, components related to each device in a health care facility.

The computing system may therefore determine, based on the location of components of a device, that a particular component is not within a predetermined proximity of the device. In this case, the computing device determines such a component to be a missing component.

In some embodiments, the alarm related to the device may comprise an indication that a device is not functioning properly. This may be, for example, an alarm in response to a status of the device being determined as not functioning properly. The alarm may be displayed as a notification or message, which may automatically be displayed in response to the device being determined as not functioning properly.

In some embodiments, the at least one action related to the device comprises a user input requesting a location of the missing component, and in response to the user input requesting a location of the missing component, the computing system is configured to locate the missing component at a location within the health care facility.

In some embodiments, the at least one action related to the device comprises a user input requesting retrieval of the missing component, and the computing system is configured to, in response to the user input requesting retrieval of the missing component, notify a health care practitioner of the healthcare facility to retrieve the missing component from the location within the health care facility. This is particularly advantageous as workflow efficiency can be improved by efficiently identifying an issue in which a component is missing, and efficiently locating the missing component such that it can be retrieved.

In some embodiments, the three-dimensional graphical representation is configured to be adjusted between a first view and a second view in response to a user input, wherein the second view represents a sub-portion of the representation of the health care facility of the first view. The first and second views may correspond to differing levels of zoom in relation to the representation of the health care facility. For example, the displayable graphical representation may first represent an entire health care facility from a street view, showing one or more buildings of the health care facility. Upon a user input, for example an input prompting a zoom of the representation, a second view of the representation may be provided. This second view may comprise a particular department of the healthcare facility such as a surgical department, showing levels of detail as described herein. Further user inputs may zoom the representation displayed to the user further, such as into a particular OR. Each view may comprise a different level of detail displayed to the user, which may include a differing level of detail of resources. For example, when displaying an entire surgical department, the displayable graphical representation may show a plurality of operating rooms, but not necessarily all patients, hospital care practitioners, and devices within particular ORs. When a particular OR is then selected by a user, more or all resources within the selected OR may be included in the graphical representation, each of which may be interactable by a user.

In some embodiments, the computing system may store information related to one or more of resources of the health care facility, patients such as patient records and vital signs, and hospital care practitioners. In some embodiments, the computing system is configured to collect data from the plurality of devices and update the resource data of the health care facility based on the collected data, and the output comprises information related to a health care facility workflow based on the collected data. The computing system may collect data, for example, from diagnostic or surgical equipment which may output data related to patient vital signs and surgical procedures. Based on the output data, the computing system may update resource data related to the functioning of one or more devices, and/or may update patient data including patient records or vital signs when receiving data from devices such as diagnostic or surgical equipment.

In some embodiments, the computing system is configured to track and display one or more of: patients; and health care workers, and the three-dimensional graphical representation is further configured to provide interactive management of the patients and/or health care workers, and preferably wherein: the interactive management comprises one or more of: viewing health care records of patients; viewing vital signs of patients; notifying health care practitioners; viewing schedules of health care practitioners; and viewing schedules of patient's appointments.

In some embodiments, the computing system is configured to communicate with, such as receive data and/or information from, a locating system such as an RTLS, the locating system being configured to track one or more of: patients; and health care workers. The computing system may send and receive data with the locating system. The data may include tracking data related to the tracking of the patients and/or health care workers. The three-dimensional graphical representation is then further configured to display, and provide interactive management of, the patients and/or health care workers based on data received from the locating system. In other words, the tracking functionality may be provided by the computing system itself, or by a software application integrated with the computing system.

The location of patients and health care practitioners may be tracked in real time in a similar manner to that described in relation to the plurality of devices in the health care facility. A user may interact with a patient and/or health care practitioner displayed by the graphical user interface in the graphical representation, for example by selecting a patient of health care practitioner. By doing so, the graphical user interface may display information associated with such a selection. In some embodiments, when a user selects a patient displayed in the graphical representation, one or more of a patient's health care records, vital signs, or schedule may be displayed. This gives a user the ability to selectively access, display, and analyse information related to the patient. The user may then access further related information. Alternatively or in addition, a user may have the ability to admit or discharge patients via user inputs through the graphical user interface. In some embodiments, the graphical user interface may provide a user with the ability to message or otherwise contact a person related to the selected patient such as a family member. Similarly, when a health care practitioner is selected, information such as the scheduled of the practitioner may be displayed. Additionally, the user may have the ability to notify or send a message to a health care practitioner.

By monitoring the location of health care practitioners, the computing system may be configured to determine when a health care practitioner is missing. For example, a computing system may have stored in its memory, information related to procedures in ORs. In relation to each procedure, the computing system may have stored information as to which health care practitioners should be present at a particular time or for a particular procedure. Through tracking the health care practitioners, it may be determined that a practitioner such as a surgeon should be present in an OR but is not present. In response to this determination, the graphical user interface may display an alarm indicating that a practitioner is missing from an OR. The computing system may be configured to automatically, or in response to a user input, notify or message a relevant health care practitioner to attend the OR. This may be a particular health care practitioner, or the relevant health care practitioner in closest proximity to the OR, determined through the tracking performed by the computing system.

In some embodiments, the system further comprises a scheduling engine being configured to collect scheduling data based on at least one of: the health care resources; monitoring of a patient in the health care facility; and monitoring a health care worker in the health care facility, and preferably wherein: the scheduling engine is further configured to output an adjustment to a schedule related to the health care facility based on the scheduling data.

In some embodiments, the computing system may be configured to communicate with, such as receive data and/or information from, a scheduling engine being configured to collect scheduling data based on at least one of: the health care resources; monitoring of a patient in the health care facility; and monitoring a health care practitioner in the health care facility. Communication may include receiving the computing system receiving scheduling data collected by the scheduling engine. The computing system is then further configured to output an adjustment to a schedule related to the health care facility based on the scheduling data received from the scheduling engine. In other words, the scheduling functionality may be provided by a software application integrated with the computing system.

The scheduling engine may therefore advantageously improve efficiency of the health care facility workflow.

In some embodiments, the system comprises one or more integrated software applications, the three-dimensional graphical representation being configured to provide interaction with the one or more integrated software applications. The integrated software applications may be configured to provide the functionality of one or more embodiments described herein. Such functionality may non-exhaustively include: the output, display, and interaction with video feeds from devices; tracking of devices, patients, and/or healthcare workers; and the functionality of the scheduling engine described herein.

According to another aspect of the present disclosure, there is provided a method for providing an interactive representation of a health care facility, the method comprising: storing, in a memory of a computing system, resource data of resources of a health care facility, the resources including a plurality of health care devices; displaying, by a graphical user interface, a three-dimensional representation of the health care facility for interactively managing the resources of the health care facility; providing, at the three-dimensional graphical representation, an output related to at least one device of the plurality of devices; and receiving, at the three-dimensional graphical representation, at least one action by a user for interacting with at least one device of the plurality of devices.

In some embodiments, the output related to the at least one device comprises one or more of: a status of the at least one device; an alarm related to the at least one device; and a video stream from the at least one device, the output being displayed by the three-dimensional graphical representation.

In some embodiments, the status of the device comprises a location status of one or more components of the device, the location status indicating whether the one or more components of the device are located within a predetermined proximity of the device.

In some embodiments, the alarm related to the device provides an indication that a component of the device is not located within the predetermined proximity of the device, and wherein the method comprises the computing system determining the component as a missing component.

In some embodiments, the at least one action related to the device comprises a user input requesting a location of the missing component, and the method further comprises, in response to the user input requesting a location of the missing component, the computing system locating the missing component at a location within the health care facility.

In some embodiments, the at least one action related to the device comprises a user input requesting retrieval of the missing component, and the method further comprises, in response to the user input requesting retrieval of the missing component, the computing system notifying a health care practitioner of the healthcare facility to retrieve the missing component from the location within the health care facility.

In some embodiments, the method further comprises three-dimensional graphical representation adjusting between a first view and a second view in response to a user input, wherein the second view represents a sub-portion of the representation of the health care facility of the first view.

In some embodiments, the method further comprises the computing system tracking the position of the plurality of devices. The tracking of the plurality of the devices may be provided by an integrated software application such as an RTLS, the computing system being in communication with the RTLS.

In some embodiments, the plurality of devices comprise one or more of: a display; a camera; a surgical table; an instrument cart; a surgical light; surgical equipment; and diagnostic equipment.

In some embodiments, the method further comprises the computing system collecting data from the plurality of devices and updating the resource data of the health care facility based on the collected data, and the output comprises information related to a health care facility workflow based on the collected data.

In some embodiments, the method further comprises the computing system communicating with a locating system, the locating system tracking one or more of: patients; and health care workers, the three-dimensional graphical representation displaying, and providing interactive management of, the patients and/or health care workers based on data received from the locating system, and preferably wherein: the interactive management comprises one or more of: viewing health care records of patients; viewing vital signs of patients; notifying health care practitioners; viewing schedules of health care practitioners; and viewing schedules of patient's appointments.

In some embodiments, the method further comprises the computing system communicating with a scheduling engine, the scheduling engine collecting scheduling data based on at least one of: the health care resources; monitoring of a patient in the health care facility; and monitoring a health care practitioner in the health care facility, and preferably wherein the method comprises the computing system outputting an adjustment to a schedule related to the health care facility based on the scheduling data received from the scheduling engine.

In some embodiments, the method further comprises the three-dimensional graphical representation interacting with one or more integrated software applications.

According to another aspect of the present disclosure, there is provided a non-transitory computer-readable medium having stored thereon instructions that, when executed, cause a processor to perform the method according to embodiments described herein.

The details of one or more examples are set forth in the accompanying drawings and the description below. Other features, objects, and advantages will be apparent from the description and drawings, and from the claims.

### BRIEF DESCRIPTION OF DRAWINGS

The invention will be described with reference to the following figures, in which:
Figure 1 is a schematic representation of a graphical user interface system according to aspects of the disclosure;
Figure 2 is a graphical representation displayed by the GUI system according to aspects of the disclosure;
Figure 3a is a portion of a graphical representation displayed by the GUI system according to aspects of the disclosure;
Figure 3b is a portion of a graphical representation displayed by the GUI system according to aspects of the disclosure;
Figure 4a is a graphical representation displayed by the GUI system according to aspects of the disclosure;
Figure 4b is a graphical representation displayed by the GUI system according to aspects of the disclosure;
Figure 5a is a portion of a graphical representation displayed by the GUI system according to aspects of the disclosure;
Figure 5b is a portion of a graphical representation displayed by the GUI system according to aspects of the disclosure;
Figure 5c is a portion of a graphical representation displayed by the GUI system according to aspects of the disclosure;
Figure 6a is a graphical representation displayed by the GUI system according to aspects of the disclosure;
Figure 6b is a graphical representation displayed by the GUI system according to aspects of the disclosure;
Figure 7a is a portion of a graphical representation displayed by the GUI system according to aspects of the disclosure;
Figure 7b is a graphical representation displayed by the GUI system according to aspects of the disclosure;
Figure 7c is a portion of a graphical representation displayed by the GUI system according to aspects of the disclosure;
Figure 8a is a graphical representation displayed by the GUI system according to aspects of the disclosure;
Figure 8b is a graphical representation displayed by the GUI system according to aspects of the disclosure;
Figure 8c is a portion of a graphical representation displayed by the GUI system according to aspects of the disclosure;
Figure 8d is a graphical representation displayed by the GUI system according to aspects of the disclosure;
Figure 9a is a graphical representation displayed by the GUI system according to aspects of the disclosure;
Figure 9b is a portion of a graphical representation displayed by the GUI system according to aspects of the disclosure;
Figure 10a is a portion of a graphical representation displayed by the GUI system according to aspects of the disclosure; and
Figure 10b is a portion of a graphical representation displayed by the GUI system according to aspects of the disclosure.

Like features are denoted by like reference numerals.

### DETAILED DESCRIPTION

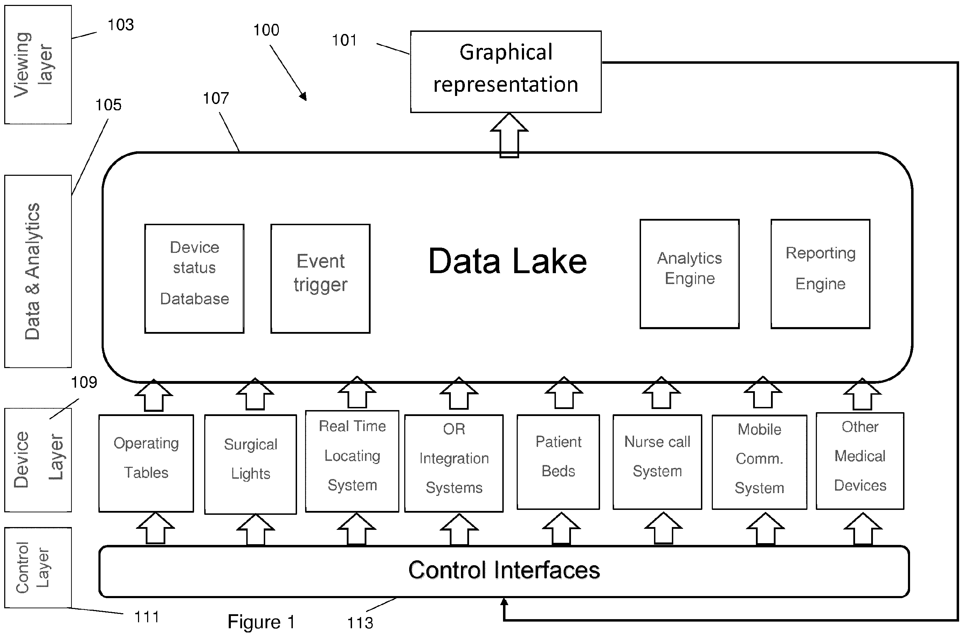

An example graphical user interface system will now be described in relation to Figures 1 to 10b.

Figure 1 illustrates an example graphical user interface system 100 according to embodiments described herein. The system 100 comprises a plurality of layers including a control layer 111, a device layer 109, a data and analytics layer 105, and a viewing layer 103, each of which will be described in detail.

The control layer 111 comprises a control interface 113. The control interface 113 enables programming and control of aspects of the computing system, including any software applications, devices, or systems integrated with the computing system. This may be performed through one or more application programming interfaces (APIs).

The device layer 109 comprises such systems and devices. In this example, the device layer 109 comprises operating tables, surgical lights, RTLS, OR integration systems, patient beds, a nurse call system, a mobile communication system, and other medical devices. The control layer is in communication connection with the device layer, which may be through a wired connection or wireless connection such as a wireless local area network (WLAN) connection. The control and programming of these devices is performed at the control layer.

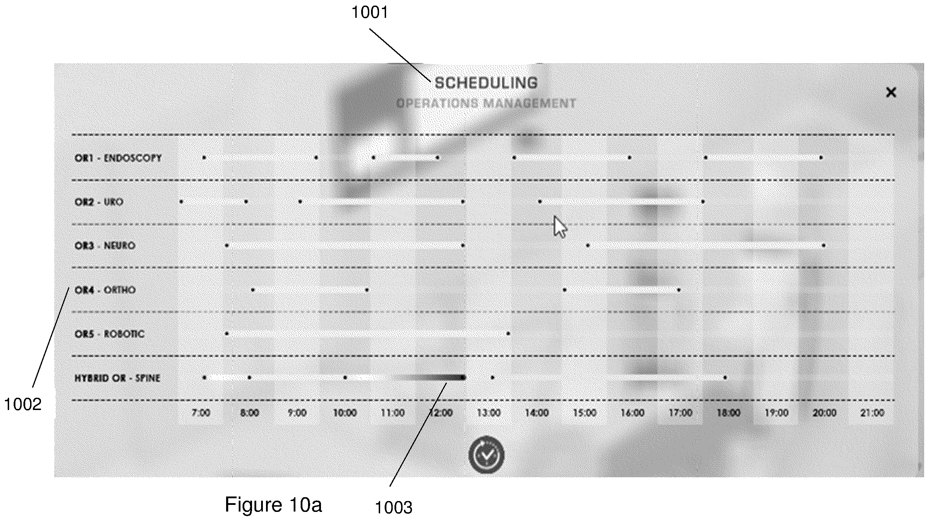

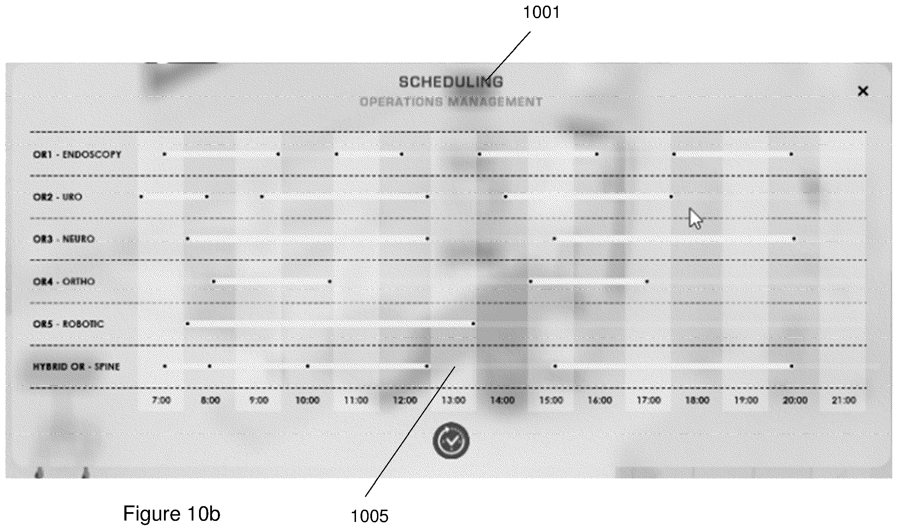

The data and analystics layer 105 may comprise the computing system according to embodiments described herein. The computing system comprises a data lake 107, which comprises elements which may be stored in the memory of the computing system. In this example, the computing system, and in particular the memory of the computing system, comprises a device status memory, information related to event triggers, an analystics engine, and a reporting engine. In this example, the analystics engine comprises the scheduling engine described in more detail with reference to Figures 10a and 10b below.

Lastly, the system 100 comprises a viewing layer 103, which in this example comprises the graphical representation 101. The graphical representation is displayable by a graphical user interface on a suitable computing device, and in this example, the graphical representation is displayed on a portable tablet computer. The data and analystics layer, and in particular the computing system, is in communication connection with the graphical representation. That is, the graphical user interface comprises a display area for displaying a graphical representation of the health care facility. The graphical representation is preferably an interactive map of the health care facility stored in the memory of the computing system.

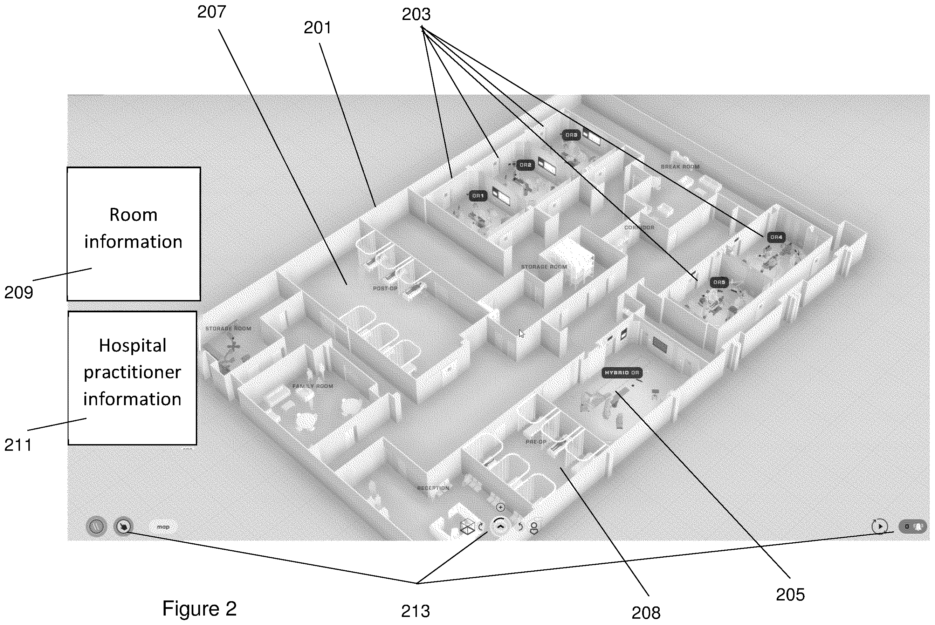

Figure 2 illustrates an example graphical representation displayed at the viewing layer 103. In this example, the graphical representation displayed by the graphical user interface on the portable tablet device is a three-dimensional graphical representation of a health care facility, and in particular a surgical department 201 of a health care facility. One or more processors of the computing system in connection with the graphical user interface provides processing for the graphical representation.

Figure 2 particularly illustrates a first view of the surgical department 201 in which the entirety of the surgical department 201 is visible at a first level of detail. For example, the graphical representation shows rooms of the surgical facility 201 including operating rooms 203, a hybrid operating room 205, a pre-op room 208, and a post-op room 207.

Visual indicators indicate the status of rooms such as operating rooms 203 and the hybrid operating room 205. In this example, colour indicators are used for a status indicating whether or not the room is in use, where a first colour is used for rooms which are in use and a second colour is used for when rooms are in use. In this example, all operating rooms are highlighted with the first colour (for example, blue) indicating that the rooms are not in use.

Figure 2 also illustrates interactive icons 213. Icons 213 provide a user of the graphical user interface to interact with, and customize, the graphical representation. In this example, icons 213 allow a user to navigate and zoom in or out the graphical representation through user inputs.

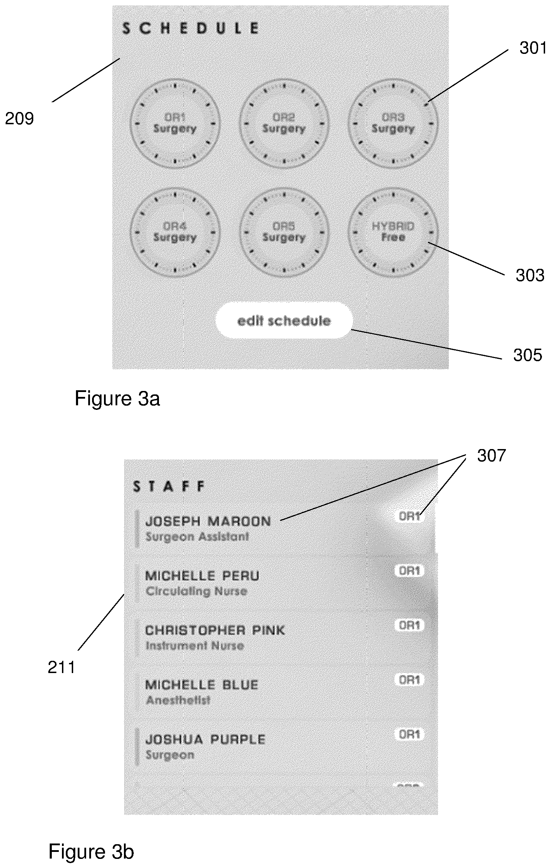

The computing system is also configured to display various information related to the health care facility through the graphical user interface. In this graphical representation view of Figure 2, room information 209 and hospital practitioner information 211 are displayed to a user, illustrated in more detail in Figures 3a and 3b respectively.

Figure 3a illustrates the room information 209 displayed through the graphical representation in Figure 2. In this example, schedule information is provided corresponding to the rooms of the surgical facility 209. The data lake or computing system in communication with the graphical user interface has stored information corresponding to the schedules or each operating room, which can be displayed at the graphical representation. A scheduling indicator 301 is displayed in relation to each operating room 203 showing the times of scheduled surgeries. A scheduling indicator 303 is displayed corresponding to the hybrid operating room 205, indicating that no surgeries are scheduled.

A user of the graphical user interface system is provided with an option to edit schedule 305. Through the communication with the computing system, the graphical representation receives an input 305 from a user, enabling the user with the ability to view and edit room schedules.

Figure 3b illustrates health care practitioner information 211 displayed through the graphical representation in Figure 2. In this example, the health care practitioner information 211 comprises a list of health care practitioners in the surgical facility 201, providing information 307 on each practitioner including their name, role, and location.

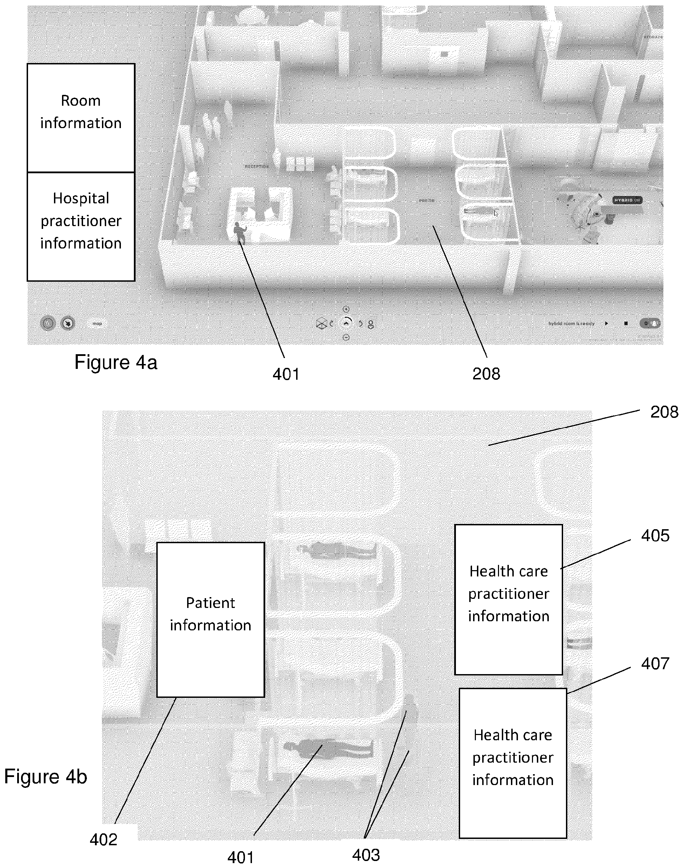

A patient journey will now be described in relation to Figures 4a to 9c.

Figure 4a illustrates a second view of the surgical facility. The second view represents a sub-portion of the first view illustrated in Figure 2. That is, in this example, the second view of Figure 4a is a zoomed in view of the first view, showing a sub-portion of the surgical facility. Here, the second view is zoomed in on the reception and pre-op 208 regions of the surgical facility 201.

A patient 401 is displayed by the graphical user interface. The patient begins their journey through the surgical facility 201 at the reception area. It is possible to track the patient 401 through the surgical facility through real-time tracking as described herein. In this example, the patient 401 is provided with an RTLS wearable device, which in this example is a wrist band, at the reception area, the wrist band being wirelessly tracked through the surgical facility. Through the RTLS, the computing system is able to track the patient 401 and display the patient 401 through communication with the graphical user interface. The RTLS tracking is provided by a software application integrated with the computing system, and the computing system received tracking data from the RTLS in order to display and provide interactive management of patients and health care workers throughout the health care facility.

Figure 4b then illustrates the patient 401 having moved to the pre-op 208 region of the surgical facility 201. The patient 401 has been tracked to the pre-op 208 region. The computing system also tracks health care practitioners 403, who in this example also wear RTLS enabling wrist bands.

Patients and health care practitioners are tracked and displayed throughout the surgical facility 201. This provides a user with the ability to interactively manage both patients and health care workers in a variety of ways.

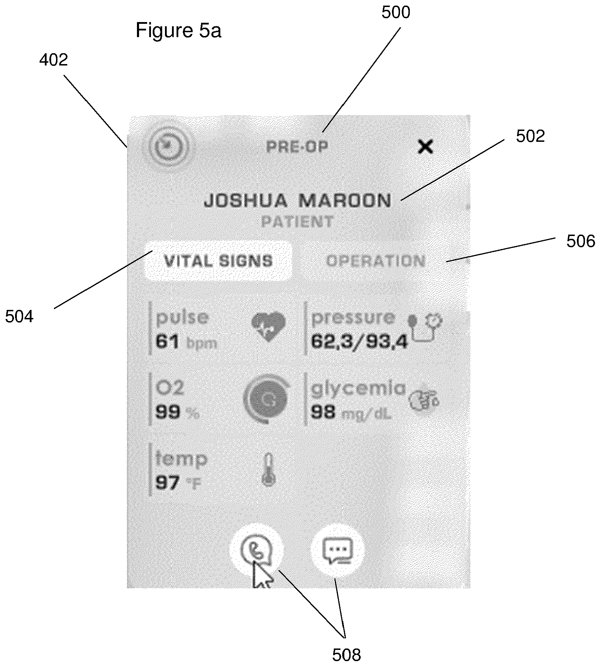

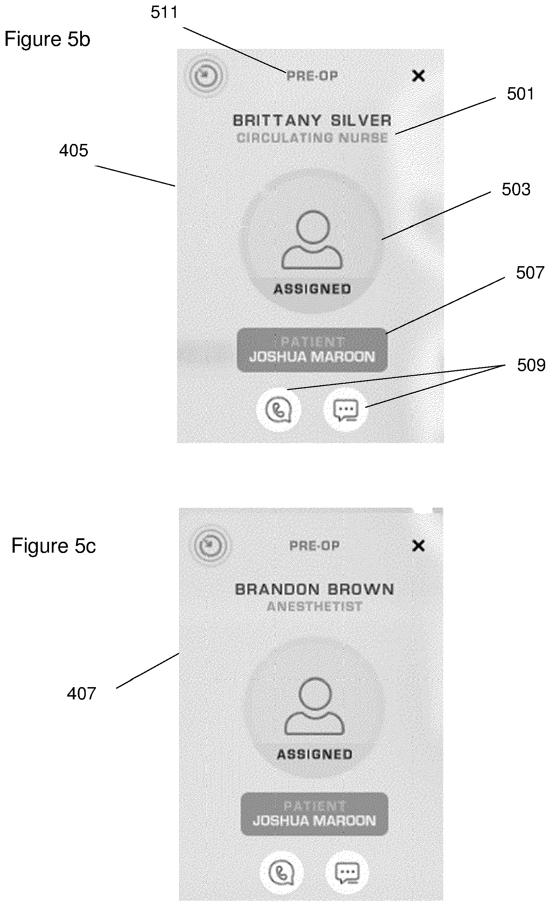

In this example, a user of the graphical user interface may select, through a touch screen input, the patient 401, which triggers the display of patient information 402, illustrated in more detail in Figure 5a.

Figure 5a illustrates the patient information 402 displayed in response to a user selection of a patient 401 through the graphical user interface. The patient information 402 is a displayable interactable pop-up overlayed on the representation of the surgical facility 201. The patient information 402 provides information including the patient name 502, the patient location 500 determined by the computing system through the integrated RTLS, a vital signs input 504 allowing the user to view the patient's vital signs, an operation input 506 allowing the user to view information related to the patient's scheduled operation, and contact inputs 508, allowing a user to contact a person or persons related to user such as a family member, which may include calling or messaging said family member. The contacting may be provided by the computer system, or an integrated software integrated with the computing system.

The vital signs are displayable at the patient information 402 through the communication between devices in the surgical department with the computing system. That is, devices such as diagnostic devices or patient beds are in wireless communication with the computing system. The devices provide outputs which are monitored and stored by the computing system, and the computing system is then configured to display the vital signs through the graphical user interface in response to the user selection of the patient 401.

Figure 4b also includes health care practitioner information, displayable in response to a user selection of the health care practitioners 403. Figure 5b and 5b illustrate the displayable health care practitioner information in more detail. In response to a user selection of the health care practitioners 403, the computing system is configured to enable display of the health care practitioner information 405, 407 through the graphical user interface.

The health care practitioner information 405 includes the health care practitioner's name and role 501, their location 511 determined by the integrated RTLS, the status of the health care practitioner 503 indicating whether or not the health care practitioner 503 is currently assigned to a task, the patient name 507, and contact inputs 509 providing the user with the ability to contact the health care practitioner 403, either by a call or message.

Figure 5b similarly illustrates another example of health care practitioner information 407 displayable by the graphical user interface.

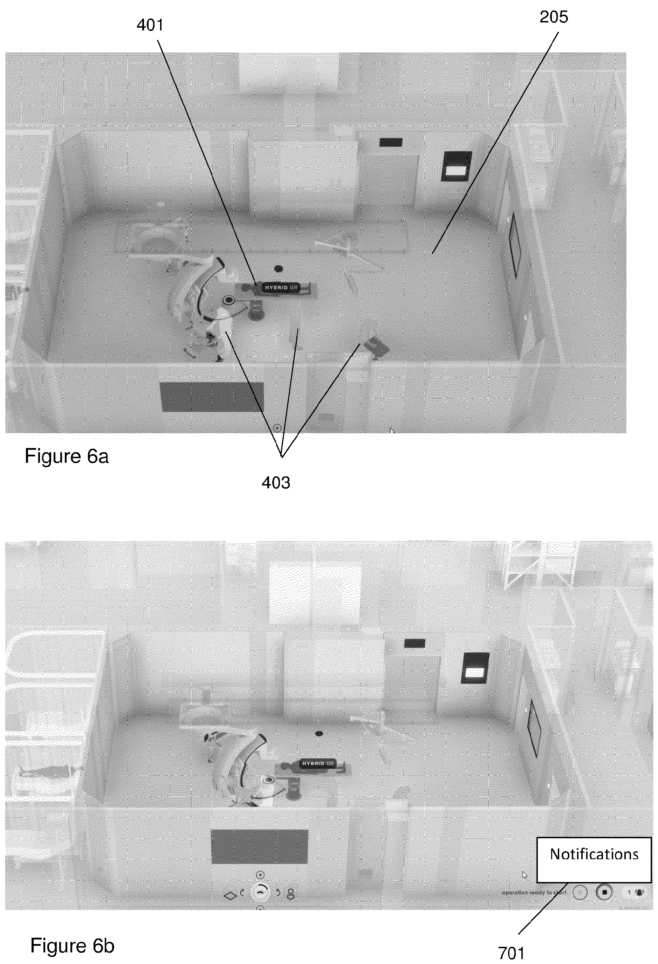

Figure 6a illustrates the next stage of the patient's journey in which another view of the surgical facility is shown. In this view, the hybrid operating room 205 is displayed. The view has been adjusted by a user through the navigational inputs such that the hybrid operating room 205 is displayed, in which the patient 401 is located on the operating table. Health care practitioners 403 are also displayed, ready to begin the patient's scheduled procedure.

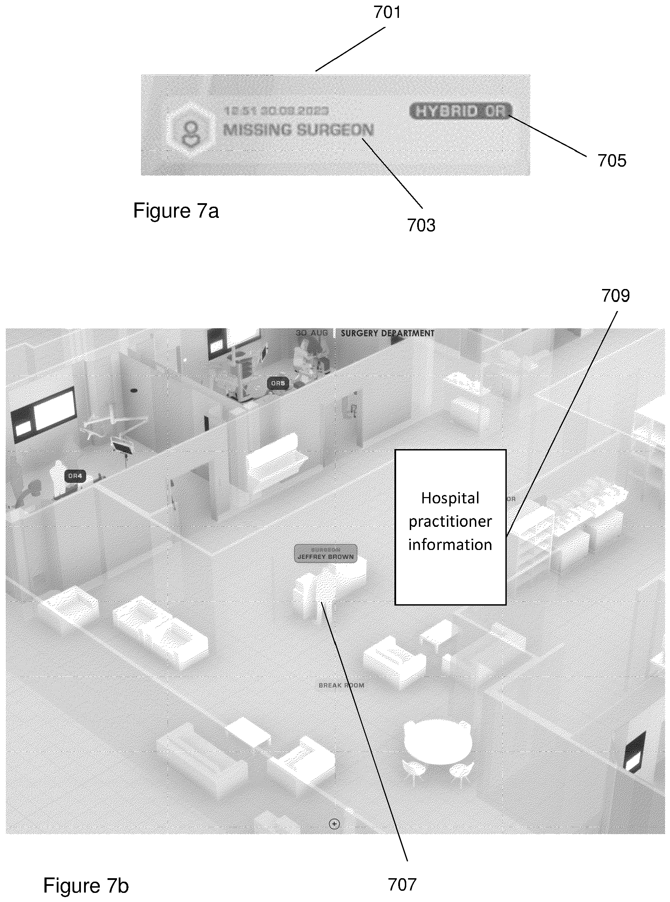

However, Figure 6b illustrates activation of an alarm through the computing system. The computing system, through data stored in a memory, determines whether the appropriate health care practitioners are present for a scheduled surgery through the integrated RTLS. In Figure 6b, the computing system determines that a health care practitioner, and in particular a surgeon, is missing from the hybrid operating room 205. In response to this determination, a notification 701 is displayed, which is illustrated more in Figure 7a.

The scheduled surgery cannot go ahead until the appropriate practitioners are present in the hybrid operating room 205. The notification 701 is an alarm output by the computing system to be displayed by the graphical user interface as illustrated in Figure 7a. The notification 701 provides information as to the cause of the alarm, which in this example is a missing surgeon 703, as well as the location of the alarm 705, which in this example is the hybrid operating room 205. The notification 701 or alarm may be displayed in a particular colour indicating an urgency of the alarm, which in this example may be a first colour such as red indicating an urgent alarm as the scheduled operation cannot go ahead without resolving the alarm.

The user may interact with the notification 701 or alarm through a touch input on the graphical user interface. Upon interaction, the computing system determines the location of the required health care practitioner through the integrated RTLS, and displays the health care practitioner 707 in their current location through the graphical user interface as illustrated in Figure 7b. Hospital practitioner information 709 is also displayed as an overlay on the graphical representation showing information related to the missing surgeon 707.

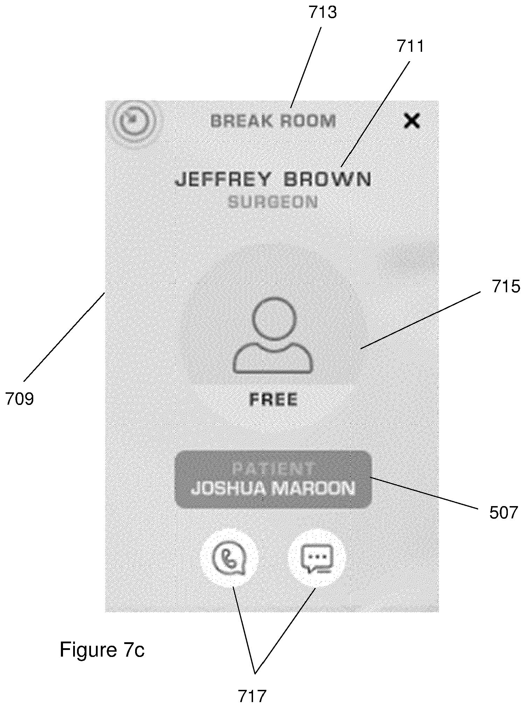

The health care practitioner information 709 is illustrated in Figure 7c, similarly to the health care practitioner information displayed as described above. The surgeons name and role 711 are displayed, as well as their location 713, the patient name 507, and contact inputs 717 enabling the user to contact the missing surgeon 707.

The user contacts the surgeon 707 through the contact inputs 717, which in this example includes messaging the surgeon 707 to attend the hybrid operating room 205 for the scheduled procedure.

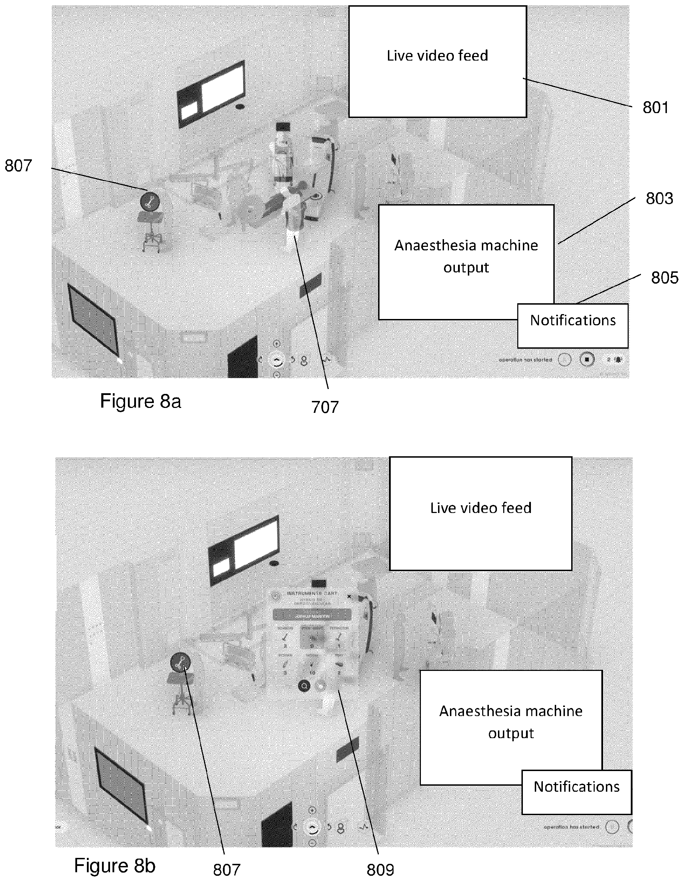

In Figure 8a, the surgeon 707 has been located and instructed to attend the hybrid operating room 205, and the required staff are therefore present. The computing system is in communication with, and interacts with, resources including the devices of the hybrid operating room 205. In this example, the devices include a surgical device comprising a video camera. The computing system monitors, can records, and is configured to display through the graphical user interface, the live video feed 801 from the surgical device, allowing a user to view the live video feed 801 through the graphical user interface. The devices also include an anaesthesia machine, and the computing system is configured to monitor, can record, and is configured to display through the graphical user interface, the output 803 from the anaesthesia machine, allowing the user to view the output 803. The live video feed 801 and output 803 are displayed as overlays over the graphical representation.

However, another notification 805 or alarm is then displayed. The computing system monitors the status of the devices in the hybrid operating room 205. The devices include a, instrument cart 807, and the computing system is configured to monitor the location of components of the instrument cart through a location status. The location status indicates the location of one or more components, and in particular, whether the components of the device are within a predetermined proximity of the instrument cart. In this way, the computing device may determine that a component of the instrument cart is not within the predetermined proximity of the device, and therefore determine that a component is missing.

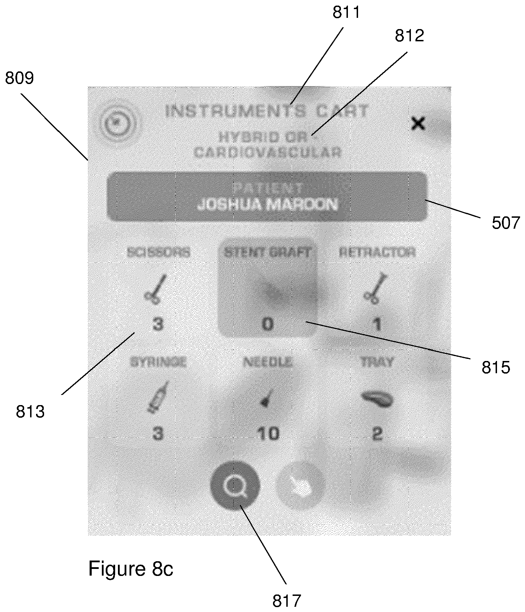

In this example, the computing system determines that an instrument is missing from the instrument cart and displays the notification 805 or alarm. In response to an interaction by the user with the notification, the computing system is configured to display, through the graphical user interface, device information 809 of the instrument cart as illustrated in Figure 8b, and illustrated in more detail in Figure 8c.

The device information 809 indicates that device type 811, the device location determined through the integrated RTLS, the patient name 507, components within the predetermined proximity 813, a component outside the predetermined proximity determined to be a missing component 815, and a search input 817. The search input is interactable by a user, allowing the user to search for the missing component in response to interacting, by a touch input, with the search input 817.

In response to an action such as a user interaction with the search input 817, the computing system determines the location of the missing component 815 through the integrated RTLS.

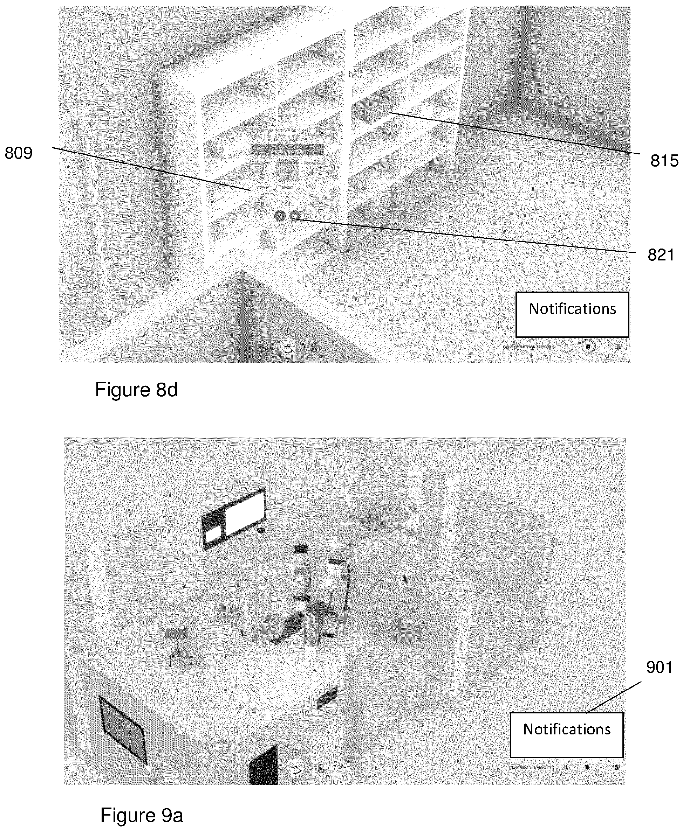

As illustrated in Figure 8d, the graphical user interface then displays the missing component 815 in its location in the surgical facility 201. In this example, the graphical user interface displays the closest, by proximity, missing component 815 in relation to the hybrid operating room 205.

Once the missing component 815 has been located, the device information provides the user with the ability to retrieve the missing component 815. In this example, a retrieval request input 821 is provided. In response to an action such as a user input, the computing system is configured to notify a health care practitioner to retrieve the missing component from the determined location. The notification to the health care practitioner may be performed through an integrated messaging system as described herein.

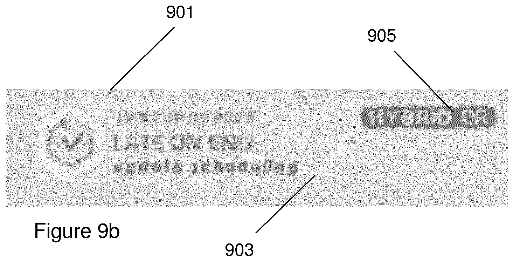

Once the missing component 815 is retrieved, the alarm is resolved and the scheduled operation may go ahead as illustrated in Figure 9a. In Figure 9a, another alarm or notification 901 is raised by the computing system and displayed by the graphical user interface, but in this example, the alarm is non-urgent, indicated to a user by a colour different to the colour of an urgent alarm. For example, the non-urgent notification or alarm, illustrated in more detail in Figure 9b, may be orange.

In this example, the non-urgent alarm or notification 901 is a scheduling alarm. Explained in more detail in relation to Figures 10a and 10b, the computing system monitors the schedules of both rooms of the surgical facility, as well as patients and hospital care workers. The computing system may perform the described scheduling, or may interact with integrated scheduling software configured to provide such functionality.

Due to the delay caused by the missing surgeon 707 and missing component 815, the patient's scheduled operation is behind schedule, indicated to the user through the alarm 901, which indicates that type of alarm 903 (in this example, a scheduling alarm), and the location of the alarm 905 indicating the hybrid operating room 205.

Figure 10a illustrates a displayable scheduling management overlay, displayable on the graphical user interface. The scheduling management overlay 1001 may be overlayed on the graphical representation, for example in response to a user interaction with the scheduling alarm 901.

As described, the computing system monitors the schedules of rooms of the surgical facility 201 through scheduling data received from the integrated scheduling software. That is, the computing system has stored in its memory, the schedules 1002 of each room including which operations are scheduled, which in this example have been downloaded through integration with the Electronic Medical record (EMR) of the health care facility. The computing system interacts with the integrated scheduling software in order to download the requested schedules.

Through the determination that the hybrid operating room 205 is behind schedule, the scheduling management overlay 1001 displays a corresponding indicator 1003, allowing the user to view a scheduling matter 1003 requiring attention.

In some embodiments, the user may manually interact with the scheduling management overlay 1001 in order to appropriately adjust the schedule to resolve the scheduling matter 1003. In response, the computing system, through interaction with the integrated scheduling software, communicates with the EMR providing a request to update the schedule, thereby providing an update to the schedule centrally.

In this example, however, the scheduling software recognizes the scheduling matter 1003 and automatically adjusts the appropriate schedule to update the matter, and interacts with the EMR to update the scheduling in the manner described. The updated scheduling management overlay is illustrated in Figure 10b, illustrating the hybrid operating rooms updated schedule 1005.

The exchange of messages between the scheduling software and the EMR may be through HL7 messaging used to transfer electronic data between healthcare systems.

In addition, the integrated scheduling software may comprise analytic functionality implemented by artificial intelligence such as machine learning. For example, the scheduling software may provide insights on data collected in relation to scheduling. The scheduling software is configured to determine patterns in scheduling such as repeated scheduling matters resulting from alarms raised. Having determined patterns in scheduling, the scheduling software is configured to automatically updated scheduling to account for predicted scheduling matters. For example, where a particular surgical procedure consistently runs behind an original schedule provided by the EMR, the scheduling software may update the schedule related to the particular surgical procedure to avoid a procedure running behind schedule and therefore avoid disruptions to the schedule. Alternatively, the scheduling software may recommend scheduling updates based on the analysis, allowing a user to provide an action such as a user input in order to manually adjust the relevant schedule according to the scheduling recommendation. The scheduling software may provide similar recommendations or automatic updates in relation to patient's and health care practitioner's schedules.

In one or more examples, the functions described may be implemented in hardware, software, firmware, or any combination thereof. If implemented in software, the functions may be stored on or transmitted over as one or more instructions or code on a computer-readable medium and executed by a hardware-based processing unit. Computer-readable media may include computer-readable storage media, which corresponds to a tangible medium such as data storage media, or communication media including any medium that facilitates transfer of a computer program from one place to another, e.g., according to a communication protocol. In this manner, computer-readable media generally may correspond to (1) tangible computer-readable storage media which is non-transitory or (2) a communication medium such as a signal or carrier wave. Data storage media may be any available media that can be accessed by one or more computers or one or more processors to retrieve instructions, code, and/or data structures for implementation of the techniques described in this disclosure. A computer program product may include a computer-readable medium.

By way of example, and not limitation, such computer-readable storage media can comprise RAM, ROM, EEPROM, CD-ROM or other optical disk storage, magnetic disk storage, or other magnetic storage devices, flash memory, or any other medium that can be used to store desired program code in the form of instructions or data structures and that can be accessed by a computer. Also, any connection is properly termed a computer-readable medium. For example, if instructions are transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. It should be understood, however, that computer-readable storage media and data storage media do not include connections, carrier waves, signals, or other transitory media, but are instead directed to non-transitory, tangible storage media. Disk and disc, as used herein, includes compact disc (CD), laser disc, optical disc, digital versatile disc (DVD), floppy disk and Blu-ray disc where disks usually reproduce data magnetically, while discs reproduce data optically with lasers. Combinations of the above should also be included within the scope of computer-readable media.

## Claims

1. A graphical user interface, GUI, system for providing an interactive representation of a health care facility, the system comprising:
a computing system comprising a memory including resource data of resources of a health care facility, the resources including a plurality of devices;
a graphical user interface in communication with the computing system; and
a three-dimensional graphical representation of the health care facility displayable by the graphical user interface for interactively managing the resources of the health care facility,
wherein the three-dimensional graphical representation is configured to provide an output related to at least one device of the plurality of devices, the output being interactable by a user for performing at least one action related to the at least one device.

2. The GUI system of claim 1, wherein the output related to the at least one device comprises one or more of: a status of the at least one device; an alarm related to the at least one device; and a video stream from the at least one device, the output being configured to be displayed by the three-dimensional graphical representation.

3. The GUI system of claim 2, wherein the status of the device comprises a location status of one or more components of the device, the location status indicating whether the one or more components of the device are located within a predetermined proximity of the device.

4. The GUI system of claim 3, wherein the alarm related to the device provides an indication that a component of the device is not located within the predetermined proximity of the device, and wherein the computing system determines the component as a missing component.

5. The GUI system of claim 4, wherein the at least one action related to the device comprises a user input requesting a location of the missing component, and in response to the user input requesting a location of the missing component, the computing system is configured to locate the missing component at a location within the health care facility.

6. The GUI system of claim 5, wherein the at least one action related to the device comprises a user input requesting retrieval of the missing component, and the computing system is configured to, in response to the user input requesting retrieval of the missing component, notify a health care practitioner of the healthcare facility to retrieve the missing component from the location within the health care facility.

7. The GUI system of any preceding claim, wherein the three-dimensional graphical representation is configured to be adjusted between a first view and a second view in response to a user input, wherein the second view represents a sub-portion of the representation of the health care facility of the first view.

8. The GUI system of any preceding claim, wherein the computing system is configured to track the position of the plurality of devices.

9. The GUI system of any preceding claim, wherein the plurality of devices comprise one or more of: a display; a camera; a surgical table; an instrument cart; a surgical light; surgical equipment; and diagnostic equipment.

10. The GUI system of any preceding claim, wherein the computing system is configured to collect data from the plurality of devices and update the resource data of the health care facility based on the collected data, and the output comprises information related to a health care facility workflow based on the collected data.

11. The GUI system of any preceding claim wherein the computing system is configured to communicate with a locating system being configured to track one or more of:
patients; and health care workers, and the three-dimensional graphical representation is further configured to display, and provide interactive management of, the patients and/or
health care workers based on data received from the locating system, and preferably wherein:
the interactive management comprises one or more of: viewing health care records of patients; viewing vital signs of patients; notifying health care practitioners; viewing schedules of health care practitioners; and viewing schedules of patient's appointments.

12. The GUI system of any preceding claim, wherein the computing system is configured to communicate with a scheduling engine being configured to collect scheduling data based on at least one of: the health care resources; monitoring of a patient in the health care facility; and monitoring a health care practitioner in the health care facility, and preferably wherein:
the computing system is further configured to output an adjustment to a schedule related to the health care facility based on the scheduling data received from the scheduling engine.

13. The GUI system of claim any preceding claim, wherein the system comprises one or more integrated software applications, the three-dimensional graphical representation being configured to provide interaction with the one or more integrated software applications.

14. A method for providing an interactive representation of a health care facility, the method comprising:
storing, in a memory of a computing system, resource data of resources of a health care facility, the resources including a plurality of health care devices;
displaying, by a graphical user interface, a three-dimensional representation of the health care facility for interactively managing the resources of the health care facility;
providing, at the three-dimensional graphical representation, an output related to at least one device of the plurality of devices; and
receiving, at the three-dimensional graphical representation, at least one action by a user for interacting with at least one device of the plurality of devices.

15. A non-transitory computer-readable medium having stored thereon instructions that, when executed, cause a processor to perform the method of claim 14.
